# EUROPEAN PATENT APPLICATION

(11) **EP 4 790 071 A2**
(43) Date of publication of application: **12.08.2026**
(21) Application number: 26179550.4
(22) Date of filing: 22.04.2021
(51) Int. Cl.: C07K 16/28, C07K 16/30, C12N 15/13, C12N 15/63, C12N 5/10, A61K 39/395, A61P 35/00, C12N 5/12

(54) **ANTI-CD73 ANTIBODY AND USE THEREOF**

(30) Priority: 22.04.2020 CN 202010324782; 23.10.2020 CN 202011152518
(62) Document number(s) of the earlier application(s) in accordance with Art. 76 EPC: 21792447.1 / 4 141 030
(71) Applicant: Akeso Biopharma, Inc., Zhongshan, Guangdong 528437 (CN)
(72) Inventor: Li, Baiyong, Zhongshan Guangdong 528437 (CN); Zhang, Peng, Zhongshan Guangdong 528437 (CN); Xia, Yu, Zhongshan Guangdong 528437 (CN); Wang, Zhongmin, Zhongshan Guangdong 528437 (CN)
(74) Representative: Ipsilon Belgium

(57) **Abstract**

Provided in the present invention are an anti-CD73 antibody and the use thereof. Specifically, a heavy chain variable region of the antibody contains HCDR1 to HCDR3 having amino acid sequences as shown in SEQ ID NOs: 15-17, respectively. Furthermore, a light chain variable region of the antibody contains LCDR1 to LCDR3 having amino acid sequences as shown in SEQ ID NOs: 18-20, respectively.

## Description

### TECHNICAL FIELD

The present invention relates to the field of immunology, and in particular to an anti-CD73 antibody and use thereof.

### BACKGROUND

Ecto-5'-nucleotidase, namely CD73 protein, is a multifunctional glycoprotein encoded by an NT5E gene and having a protein molecular weight of 70 KD, which is anchored on a cell membrane by glycosylphosphatidylinositol (GPI) (Zimmermann H. Biochem J. 1992; 285:345-365).

CD73 is widely distributed on the surface of human tissue cells, and it has been found in research that CD73 is highly expressed in various solid tumors, specifically in cancer cells, dendritic cells, regulatory T cells (Tregs), natural killer cells (NK cells), myeloid-derived suppressor cells (MDSCs), tumor-associated macrophages (TAMs) and the like in a tumor microenvironment. An important feature of the tumor microenvironment is hypoxia. Hypoxia induces the up-regulation of molecules such as hypoxia-inducible factor-1 (HIF-1), thereby leading to the widespread expression of CD73 in the tumor microenvironment (Synnestvedt K, et al. J Clin Invest. 2002; 110:993-1002). Analysis of clinical tumor samples shows that high expression of CD73 is a potential biomarker and is closely related to adverse prognosis of various types of tumors, including breast cancer, lung cancer, ovarian cancer, kidney cancer, gastric cancer, head and neck cancer and the like.

CD73 has both hydrolase activity and non-hydrolase activity. The enzyme and non-enzyme functions of CD73 are simultaneously present in the related process of tumor, and mutually promote and maintain the evolution of tumor. More and more studies have found that CD73 is a key regulatory molecule for tumor cell proliferation, metastasis and invasion *in vitro*, and tumor angiogenesis and tumor immune escape mechanism *in vivo*, wherein an important immune suppression mechanism is mediated by CD73-adenosine metabolic signal pathway. CD39 at the upstream of CD73 can catalyze ATP to generate adenosine monophosphate (AMP), the generated AMP is converted into adenosine by CD73, and adenosine binds to a downstream adenosine receptor (A2AR). A2AR inhibits a series of signal pathways related to immune activation, such as LCK, MAPK, PKC, and inhibits the immune killing effect of T cells by activating protein kinase A (PKA) and Csk kinase, thereby playing an immune suppression role to enable tumor to achieve immune escape (Antonioli L, et al. Nat Rev Cancer. 2013; 13:842-857). Preclinical animal model studies show that CD73 expressed by immune cells and non-immune cells can promote the immune escape, development and metastasis of tumors, wherein the inhibition of cytotoxic T cell (CTL) and NK cell functions by Treg cell-related CD73-adenosine signals is most obvious.

For the treatment of solid tumors, one important aspect to overcome drug resistance and improve the curative effect is to relieve the inhibition effect of the tumor microenvironment (TME) on immune effector cells. TME is a very complex system composed of various cells, intercellular matrix, enzymes, cytokines, metabolites, etc., and it has the characteristics of significant low hydrogen, low pH and high pressure, and is very different from normal tissues. Hypoxia or ATP enrichment caused by chemoradiotherapy for killing tumor cells promotes the cascade reaction of CD39-CD73 adenosine signals, which is beneficial to the proliferation and function of various cancer-promoting cells, and is not beneficial to cancer-inhibiting cells (Regateiro, F. S., Cobbold, S. P. & Waldmann, H. Clin. Exp. Immunol. 2013; 171:1-7).

The use of antibodies targeting CD73 or gene knock-out of CD73 in animal models can effectively block the growth and metastasis of tumors. Recently, the use of CD73 monoclonal antibody, small interfering RNA technology, specific inhibitor APCP and the like has achieved remarkable curative effect in anti-tumor treatment of animal experiments, providing a new way for anti-tumor treatment. Evidence from *in vivo* experiments shows that targeted blockade of CD73 will be an effective treatment means for tumor patients.

The relation between CD73 over-expression and tumor subtype, prognosis and drug response of patients has shown that CD73 can be an important marker for future tumor treatment and detection of individuals. Therefore, the study of the CD73 target is indispensable.

### SUMMARY

After intensive studies and creative efforts, the inventor used mammalian cell expression systems to express recombinant human CD73 as an antigen to immunize mice, and obtained hybridoma cells by fusion of mouse spleen cells and myeloma cells. The inventor obtained a hybridoma cell line LT014 (preservation number: CCTCC NO: C2018137) by screening a large number of samples.

The inventor surprisingly has found that the hybridoma cell line LT014 can secrete a specific monoclonal antibody (named as 19F3) specifically binding to human CD73, and the monoclonal antibody can effectively inhibit the enzyme activity reaction of CD73 in a non-substrate competition mode, reduce the production of adenosine, promote the activity of T cells, and exert the effect of inhibiting tumor growth. Further, the inventor has creatively produced humanized antibodies against human CD73 (named as 19F3H1L1, 19F3H2L2 and 19F3H2L3); and on the basis of the above-described humanized antibodies, a novel humanized antibody with ADCC activity eliminated (named as 19F3H1L1(hG1DM), 19F3H2L2(hG1DM) and 19F3H2L3(hG1DM)) was obtained by introducing an amino acid mutation into the heavy chain constant region.

The inventor has also surprisingly found that the antibodies of the present invention, such as 19F3H1L1, 19F3H2L2 and 19F3H2L3, have the activity of inducing endocytosis of CD73 expressed by cell membranes, and can reduce the activity of CD73, and further mutated antibodies, such as 19F3H2L3(hG1DM), do not bind to FcγRIIIa which causes antibody-mediated cytotoxicity, thereby significantly improving safety. The antibodies of the present invention have potential for use in the treatment of tumors.

One aspect of the present invention relates to an anti-CD73 (for example, human CD73) antibody or an antigen-binding fragment thereof, wherein the anti-CD73 antibody comprises HCDR1, HCDR2 and HCDR2 contained in a heavy chain variable region set forth in SEQ ID NO: 2, SEQ ID NO: 6 or SEQ ID NO: 10; and LCDR1, LCDR2 and LCDR3 contained in a light chain variable region set forth in SEQ ID NO: 4, SEQ ID NO: 8, SEQ ID NO: 12 or SEQ ID NO: 14, preferably, according to an IMGT numbering system, the anti-CD73 antibody comprises:
HCDR1, comprising or consisting of an amino acid sequence set forth in SEQ ID NO: 15, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2 or 3) conservative amino acid mutations (preferably substitutions, insertions or deletions) compared to the sequence, HCDR2, comprising or consisting of an amino acid sequence set forth in SEQ ID NO: 16, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2 or 3) conservative amino acid mutations (preferably substitutions, insertions or deletions) compared to the sequence, HCDR3, comprising or consisting of an amino acid sequence set forth in SEQ ID NO: 17, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2 or 3) conservative amino acid mutations (preferably substitutions, insertions or deletions) compared to the sequence, LCDR1, comprising or consisting of an amino acid sequence set forth in SEQ ID NO: 18, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2 or 3) conservative amino acid mutations (preferably substitutions, insertions or deletions) compared to the sequence, LCDR2, comprising or consisting of an amino acid sequence set forth in SEQ ID NO: 19, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2 or 3) conservative amino acid mutations (preferably substitutions, insertions or deletions) compared to the sequence, and
LCDR3, comprising or consisting of an amino acid sequence set forth in SEQ ID NO: 20, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2 or 3) conservative amino acid mutations (preferably substitutions, insertions or deletions) compared to the sequence. In some embodiments of the present invention, the heavy chain variable region of the antibody comprises or consists of the following sequences:
a sequence set forth in SEQ ID NO: 2, SEQ ID NO: 6 or SEQ ID NO: 10, a sequence having at least 80%, 81%, 82%, 83%, 84%, 85%, 86%, 87%, 88%, 89%, 90%, 91%, 92%, 93%, 94%, 95%, 96%, 97%, 98% or 99% sequence identity to the sequence set forth in SEQ ID NO: 2, SEQ ID NO: 6 or SEQ ID NO: 10, or an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 2, SEQ ID NO: 6 or SEQ ID NO: 10; and
the light chain variable region of the antibody comprises or consists of the following sequences:
   a sequence set forth in SEQ ID NO: 4, SEQ ID NO: 8, SEQ ID NO: 12 or SEQ ID NO: 14, a sequence having at least 80%, 81%, 82%, 83%, 84%, 85%, 86%, 87%, 88%, 89%, 90%, 91%, 92%, 93%, 94%, 95%, 96%, 97%, 98% or 99% sequence identity to the sequence set forth in SEQ ID NO: 4, SEQ ID NO: 8, SEQ ID NO: 12 or SEQ ID NO: 14, or an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 4, SEQ ID NO: 8, SEQ ID NO: 12 or SEQ ID NO: 14.

In some embodiments of the present invention, the heavy chain variable region of the antibody comprises an amino acid sequence set forth in SEQ ID NO: 2, and the light chain variable region of the antibody comprises an amino acid sequence set forth in SEQ ID NO: 4;
the heavy chain variable region of the antibody comprises an amino acid sequence set forth in SEQ ID NO: 6, and the light chain variable region of the antibody comprises an amino acid sequence set forth in SEQ ID NO: 8;
the heavy chain variable region of the antibody comprises an amino acid sequence set forth in SEQ ID NO: 10, and the light chain variable region of the antibody comprises an amino acid sequence set forth in SEQ ID NO: 12; or
the heavy chain variable region of the antibody comprises an amino acid sequence set forth in SEQ ID NO: 10, and the light chain variable region of the antibody comprises an amino acid sequence set forth in SEQ ID NO: 14.

In some embodiments of the present invention, the antibody comprises a heavy chain constant region having a sequence set forth in SEQ ID NO: 21, that is, a leucine to alanine point mutation is introduced at position 234 (L234A) and a leucine to alanine point mutation is introduced at position 235 (L235A) based on Ig gamma-1 chain C region and ACCESSION: P01857, and a light chain constant region having a sequence set forth in SEQ ID NO: 22, namely Ig kappa chain C region, ACCESSION: P01834; or the heavy chain constant region is Ig gamma-1 chain C region, ACCESSION: P01857, and the light chain constant region is Ig kappa chain C region, ACCESSION: P01834).

The variable regions of the light chain and the heavy chain determine the binding of the antigen; the variable region of each chain contains three hypervariable regions called complementarity determining regions (CDRs) (CDRs of the heavy chain (H) comprise HCDR1, HCDR2 and HCDR3, and CDRs of the light chain (L) comprise LCDR1, LCDR2 and LCDR3, which are named by Kabat et al., see Bethesda M.d., Sequences of Proteins of Immunological Interest, Fifth Edition, NIH Publication 1991; 1-3:91-3242.

Preferably, CDRs may also be defined by the IMGT numbering system, see Ehrenmann, Francois, Quentin Kaas, and Marie-Paule Lefranc. IMGT/3Dstructure-DB and IMGT/DomainGapAlign: a database and a tool for immunoglobulins or antibodies, T cell receptors, MHC, IgSF and MhcSF. Nucleic acids research 2009; 38(suppl_1): D301-D307.

The amino acid sequences of the CDR regions of the monoclonal antibody sequences are analyzed according to the IMGT definition by technical means well known to those skilled in the art, for example by using the VBASE2 database.

The antibodies 19F3, 19F3H1L1, 19F3H2L2 and 19F3H2L3 involved in the present invention have identical CDRs.

The amino acid sequences of the 3 CDR regions of the heavy chain variable region are as follows:
HCDR1: GYSFTGYT (SEQ ID NO: 15),
HCDR2: INPYNAGT (SEQ ID NO: 16), and
HCDR3: ARSEYRYGGDYFDY (SEQ ID NO: 17);
the amino acid sequences of the 3 CDR regions of the light chain variable region are as follows:
   LCDR1: QSLLNSSNQKNY (SEQ ID NO: 18),
   LCDR2: FAS (SEQ ID NO: 19), and
   LCDR3: QQHYDTPYT (SEQ ID NO: 20).

In some embodiments of the present invention, the antibody is a monoclonal antibody.

In some embodiments of the present invention, the antibody is a humanized antibody, a chimeric antibody or a multispecific antibody (e.g., a bispecific antibody).

In some embodiments of the present invention, the antigen-binding fragment is selected from Fab, Fab', F(ab')₂, Fd, Fv, dAb, Fab/c, a complementarity determining region fragment, a single chain antibody (e.g., scFv), a humanized antibody, a chimeric antibody and a bispecific antibody. Another aspect of the present invention relates to an isolated polypeptide selected from the group consisting of:
(1) an isolated polypeptide, comprising sequences set forth in SEQ ID NO: 15, SEQ ID NO: 16 and SEQ ID NO: 17, wherein the polypeptide specifically binds to CD73 as a part of an anti-CD73 antibody, and the antibody further comprises sequences set forth in SEQ ID NO: 18, SEQ ID NO: 19 and SEQ ID NO: 20;
(2) an isolated polypeptide, comprising sequences set forth in SEQ ID NO: 18, SEQ ID NO: 19 and SEQ ID NO: 20, wherein the polypeptide specifically binds to CD73 as a part of an anti-CD73 antibody, and the antibody further comprises sequences set forth in SEQ ID NO: 15, SEQ ID NO: 16 and SEQ ID NO: 17;
(3) an isolated polypeptide, comprising a sequence set forth in SEQ ID NO: 2, SEQ ID NO: 6 or SEQ ID NO: 10, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence, wherein the polypeptide specifically binds to CD73 as a part of an anti-CD73 antibody, and the antibody further correspondingly comprises a sequence set forth in SEQ ID NO: 4, SEQ ID NO: 8 or SEQ ID NO: 12, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence;
(4) an isolated polypeptide, comprising a sequence set forth in SEQ ID NO: 4, SEQ ID NO: 8 or SEQ ID NO: 12, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence, wherein the polypeptide specifically binds to CD73 as a part of an anti-CD73 antibody, and the antibody further correspondingly comprises a sequence set forth in SEQ ID NO: 2, SEQ ID NO: 6 or SEQ ID NO: 10, a sequence having at least 80% to the sequence, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence;
(5) an isolated polypeptide, comprising a sequence set forth in SEQ ID NO: 10, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence, wherein the polypeptide specifically binds to CD73 as a part of an anti-CD73 antibody, and the antibody further correspondingly comprises a sequence set forth in SEQ ID NO: 14, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence; and
(6) an isolated polypeptide, comprising a sequence set forth in SEQ ID NO: 14, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence, wherein the polypeptide specifically binds to CD73 as a part of an anti-CD73 antibody, and the antibody further correspondingly comprises a sequence set forth in SEQ ID NO: 10, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence. Another aspect of the present invention relates to an isolated nucleic acid molecule encoding the antibody or the antigen-binding fragment thereof or the isolated polypeptide according to any one of the aspects of the present invention.

Yet another aspect of the present invention relates to a vector comprising the isolated nucleic acid molecule disclosed herein.

Yet another aspect of the present invention relates to a host cell comprising the isolated nucleic acid molecule or the vector disclosed herein.

Yet another aspect of the present invention relates to a conjugate comprising an antibody and a conjugated moiety, wherein the antibody is the antibody or the antigen-binding fragment thereof according to any one of the aspects of the present invention,, and the conjugated moiety is a purification tag (e.g., a His tag), a detectable label; preferably, the conjugated moiety is a radioisotope, a fluorescent substance, a chemiluminescent substance, a colored substance, polyethylene glycol or an enzyme.

Yet another aspect of the present invention relates to a fusion protein or a multispecific antibody (preferably a bispecific antibody) comprising the antibody or the antigen-binding fragment thereof according to any one of the aspects of the present invention.

Yet another aspect of the present invention relates to a kit comprising the antibody or the antigen-binding fragment thereof according to any one of the aspects of the present invention, the conjugate, the fusion protein or the multispecific antibody of the present invention; preferably, the kit further comprises a secondary antibody specifically recognizing the antibody; optionally, the secondary antibody further comprises a detectable label, such as a radioisotope, a fluorescent substance, a chemiluminescent substance, a colored substance or an enzyme.

Yet another aspect of the present invention relates to use of the antibody or the antigen-binding fragment thereof according to any one of the aspects of the present invention, the conjugate, the fusion protein or the multispecific antibody of the present invention in preparing a kit used for detecting the presence or level of CD73 in a sample.

Yet another aspect of the present invention relates to a pharmaceutical composition comprising the antibody or the antigen-binding fragment thereof according to any one of the aspects of the present invention, the conjugate, the fusion protein or the multispecific antibody of the present invention; optionally, the pharmaceutical composition further comprises a pharmaceutically acceptable carrier and/or excipient. Preferably, the pharmaceutical composition is in a form suitable for administration by subcutaneous injection, intradermal injection, intravenous injection, intramuscular injection or intralesional injection.

Yet another aspect of the present invention relates to use of the antibody or the antigen-binding fragment thereof according to any one of the aspects of the present invention, the conjugate, the fusion protein or the multispecific antibody of the present invention in preparing drugs for treating and/or preventing a tumor (such as a solid tumor, preferably non-small cell lung cancer, prostate cancer (including metastatic castration-resistant prostate cancer (mCRPC)), triple-negative breast cancer, ovarian cancer, colorectal cancer (including colorectal cancer microsatellite stability (MSS)), gastric cancer, melanoma, head and neck cancer, renal cell carcinoma or pancreatic ductal adenocarcinoma), or in preparing a drug for diagnosing a tumor.

Yet another aspect of the present invention relates to a hybridoma cell line LT014, which was deposited at China Center for Type Culture Collection (CCTCC) with a collection number of CCTCC NO: C2018137.

In the present invention, unless otherwise specified, the scientific and technical terms used herein have the meanings generally understood by those skilled in the art. In addition, the laboratory operations of cell culture, molecular genetics, nucleic acid chemistry and immunology used herein are the routine procedures widely used in the corresponding fields. Meanwhile, in order to better understand the present invention, the definitions and explanations of the relevant terms are provided below.

As used herein, the term EC₅₀ refers to the concentration for 50% of maximal effect, i.e., the concentration that can cause 50% of the maximal effect.

As used herein, the term "antibody" refers to an immunoglobulin molecule that generally consists of two pairs of polypeptide chains (each pair with one "light" (L) chain and one "heavy" (H) chain). Antibody light chains are classified as κ and λ light chains. Heavy chains are classified as µ, δ, γ, α, or ε. Isotypes of antibodies are defined as IgM, IgD, IgG, IgA, and IgE. In light chains and heavy chains, the variable region and constant region are linked by a "J" region of about 12 or more amino acids, and the heavy chain also comprises a "D" region of about 3 or more amino acids. Each heavy chain consists of a heavy chain variable region (V_{H}) and a heavy chain constant region (C_{H}). The heavy chain constant region consists of 3 domains (C_{H1}, C_{H2}, and C_{H3}). Each light chain consists of a light chain variable region (V_{L}) and a light chain constant region (C_{L}). The light chain constant region consists of one domain C_{L}. The constant region of the antibody can mediate the binding of immunoglobulins to host tissues or factors, including various cells of the immune system (e.g., effector cells) and the first component (C1q) of the classical complement system. The V_{H} and V_{L} regions can be further subdivided into highly variable regions (called complementarity determining regions (CDRs)), between which conservative regions called framework regions (FRs) are distributed. Each V_{H} and V_{L} consists of 3 CDRs and 4 FRs arranged from amino terminus to carboxyl terminus in the following order: FR1, CDR1, FR2, CDR2, FR3, CDR3, FR4. The variable regions (V_{H} and V_{L}) of each heavy chain/light chain pair form antigen-binding sites, respectively. The assignment of amino acids to the regions or domains is based on Kabat Sequences of Proteins of Immunological Interest (National Institutes of Health, Bethesda, M.d. (1987 and 1991)), or Chothia & Lesk J. Mol. Biol. 1987; 196:901-917; Chothia et al. Nature 1989; 342:878-883 or the definition of the IMGT numbering system, see the definition in Ehrenmann, Francois, Quentin Kaas, and Marie-Paule Lefranc. IMGT/3Dstructure-DB and IMGT/DomainGapAlign: a database and a tool for immunoglobulins or antibodies, T cell receptors, MHC, IgSF and MhcSF. Nucleic acids research 2009; 38(suppl_1): D301-D307. The term "antibody" is not limited by any specific method for producing antibody. For example, the antibody includes, in particular, a recombinant antibody, a monoclonal antibody, and a polyclonal antibody. The antibody may be antibodies of different isotypes, such as IgG (e.g., subtype IgG1, IgG2, IgG3 or IgG4), IgA1, IgA2, IgD, IgE or IgM.

As used herein, the terms "mAb" and "monoclonal antibody" refer to an antibody or a fragment of an antibody that is derived from a group of highly homologous antibodies, i.e., from a group of identical antibody molecules, except for natural mutations that may occur spontaneously. The monoclonal antibody is highly specific for a single epitope on an antigen. The polyclonal antibody, relative to the monoclonal antibody, generally comprises at least 2 or more different antibodies which generally recognize different epitopes on an antigen. Monoclonal antibodies can generally be obtained using hybridoma technology first reported by Kohler et al (Köhler G, Milstein C. Continuous cultures of fused cells secreting antibody of predefined specificity [J]. Nature, 1975; 256(5517): 495), but can also be obtained using recombinant DNA technology (see, e.g., U.S. Patent 4,816,567).

As used herein, the term "humanized antibody" refers to an antibody or an antibody fragment obtained when all or a part of CDR regions of a human immunoglobulin (receptor antibody) are replaced by the CDR regions of a non-human antibody (donor antibody), wherein the donor antibody may be a non-human (e.g., mouse, rat or rabbit) antibody having expected specificity, affinity or reactivity. In addition, some amino acid residues in the framework regions (FRs) of the receptor antibody can also be replaced by the amino acid residues of corresponding non-human antibodies or by the amino acid residues of other antibodies to further improve or optimize the performance of the antibody. For more details on humanized antibodies, see, e.g., Jones et al., Nature 1986; 321:522-525; Reichmann et al., Nature 1988; 332:323-329; Presta, Curr. Op. Struct. Biol., 1992; 2:593-596; and Clark M. Antibody humanization: a case of the 'Emperor's new clothes'? [J]. Immunol. Today, 2000; 21(8): 397-402.

As used herein, the term "isolated" refers to obtaining by artificial means from natural state. If a certain "isolated" substance or component appears in nature, it may be the case that change occurs in its natural environment, or that it is isolated from the natural environment, or both. For example, a certain non-isolated polynucleotide or polypeptide naturally occurs in a certain living animal, and the same polynucleotide or polypeptide with high purity isolated in such a natural state is referred to as an isolated polynucleotide or polypeptide. The term "isolated" does not exclude the existence of artificial or synthetic substances or other impurities that do not affect the activity of the substance.

As used herein, the term "vector" refers to a nucleic acid vehicle into which a polynucleotide can be inserted. When a vector allows for the expression of the protein encoded by the inserted polynucleotide, the vector is referred to as an expression vector. The vector can be introduced into a host cell by transformation, transduction, or transfection so that the genetic substance elements carried by the vector can be expressed in the host cell. Vectors are well known to those skilled in the art, including but not limited to: plasmids; phagemids; cosmids; artificial chromosomes, such as yeast artificial chromosome (YAC), bacterial artificial chromosome (BAC), or P1-derived artificial chromosome (PAC); phages such as lambda phages or M13 phages; and animal viruses. Animal viruses that can be used as vectors include, but are not limited to retroviruses (including lentiviruses), adenoviruses, adeno-associated viruses, herpes viruses (such as herpes simplex virus), poxviruses, baculoviruses, papillomaviruses, and papovaviruses (such as SV40). A vector may comprise a variety of elements that control expression, including, but not limited to promoter sequences, transcription initiation sequences, enhancer sequences, selection elements, and reporter genes. In addition, the vector may further comprise a replication initiation site.

As used herein, the term "host cell" refers to cells to which vectors can be introduced, including, but not limited to, prokaryotic cells such as *E. coli* or *bacillus subtilis*, fungal cells such as yeast cells or *aspergillus*, insect cells such as S2 drosophila cells or Sf9, or animal cells such as fibroblasts, CHO cells, COS cells, NSO cells, HeLa cells, GS cells, BHK cells, HEK 293 cells, or human cells.

As used herein, the term "specific binding" refers to a non-random binding reaction between two molecules, such as a reaction between an antibody and an antigen it targets. In some embodiments, an antibody specifically binding to an antigen (or an antibody specific to an antigen) means that the antibody binds to the antigen with an affinity (K_{D}) of less than about 10⁻⁵ M, e.g., less than about 10⁻⁶ M, 10⁻⁷ M, 10⁻⁸ M, 10⁻⁹ M or 10⁻¹⁰ M or less.

As used herein, the term "K_{D}" refers to a dissociation equilibrium constant for a specific antibody-antigen interaction, which is used to describe the binding affinity between the antibody and the antigen. A smaller dissociation equilibrium constant indicates a stronger antibody-antigen binding and a higher affinity between the antibody and the antigen. Generally, antibodies bind to antigens (e.g., PD-1 protein) with a dissociation equilibrium constant (K_{D}) of less than about 10⁻⁵ M, e.g., less than about 10⁻⁶ M, 10⁻⁷ M, 10⁻⁸ M, 10⁻⁹ M or 10⁻¹⁰ M or less. K_{D} can be determined using methods known to those skilled in the art, e.g., using a Fortebio system.

As used herein, the terms "monoclonal antibody" and "mAb" have the same meaning and can be used interchangeably; the terms "polyclonal antibody" and "pAb" have the same meaning and can be used interchangeably; the terms "polypeptide" and "protein" have the same meaning and can be used interchangeably. Besides, herein, amino acids are generally represented by single-letter and three-letter abbreviations known in the art. For example, alanine can be represented by A or Ala.

As used herein, the term "pharmaceutically acceptable carrier and/or excipient" refers to a carrier and/or excipient that is pharmacologically and/or physiologically compatible with the subject and the active ingredient. Such carriers and/or excipients are well known in the art (see, e.g., Remington's Pharmaceutical Sciences, edited by Gennaro AR, 19th Ed., Pennsylvania: Mack Publishing Company, 1995), including, but not limited to: pH regulators, surfactants, adjuvants, and ionic strength enhancers. For example, the pH regulators include, but are not limited to, phosphate buffer; the surfactants include, but are not limited to, cationic, anionic, or non-ionic surfactants, such as Tween-80; the ionic strength enhancers include, but are not limited to, sodium chloride.

As used herein, the term "effective amount" refers to an amount sufficient to obtain or at least partially obtain desired effects. For example, a prophylactically effective amount against a disease (e.g., a tumor) refers to an amount sufficient to prevent, stop, or delay the onset of a disease (e.g., a tumor); a therapeutically effective amount refers to an amount sufficient to cure or at least partially stop a disease and complications thereof in patients suffering from the disease.

Beneficial effects of the present invention:
the monoclonal antibody of the present invention can specifically bind to CD73 well, and can effectively inhibit the enzyme activity reaction of CD73 in a non-substrate competition mode, reduce the production of adenosine, and promote the activity of T cells and the tumor inhibitory effect.

Notes on the deposit of biological materials:
the hybridoma cell line LT014 (also called CD73-19F3) was deposited at China Center for Type Culture Collection (CCTCC) on June 21, 2018 with a collection number of CCTCC NO: C2018137 and a collection address of Wuhan University, Wuhan, China, postal code: 430072.

### BRIEF DESCRIPTION OF THE DRAWINGS

FIG. 1. Detection results of affinity constants of 19F3H2L3 for HNT5E(1-552)-his. The antibody concentrations for the curve pairs from top to bottom are 200 nM, 100 nM, 50 nM, 25 nM, 12.5 nM, 6.25 nM and 3.125 nM, respectively.
FIG. 2. Detection results of affinity constants of 19F3H2L2 for HNT5E(1-552)-his. The antibody concentrations for the curve pairs from top to bottom are 200 nM, 100 nM, 50 nM, 25 nM, 12.5 nM, 6.25 nM and 3.125 nM, respectively.
FIG. 3. Detection results of affinity constants of MEDI9447 for HNT5E(1-552)-his. The antibody concentrations for the curve pairs from top to bottom are 200 nM, 100 nM, 50 nM, 25 nM, 12.5 nM, 6.25 nM and 3.125 nM, respectively.
FIG. 4. Binding activity of 19F3H2L3 to CD73 on the surface of MDA-MB-231 cells determined by FACS.
FIG. 5. Detection results of enzyme activity of anti-CD73 antibody added to MDA-MB-231 cells.
FIG. 6. Detection results of enzyme activity of anti-CD73 antibody added to U87-MG cells.

### DETAILED DESCRIPTION

The embodiments of the present invention will be described in detail below with reference to the examples. Those skilled in the art will understand that the following examples are only for illustrating the present invention, and should not be construed as limitations on the scope of the present invention. In the cases where the techniques or conditions are not specified, the examples were implemented according to the techniques or conditions described in the literature in the art (e.g., see, Molecular Cloning: A Laboratory Manual, authored by J. Sambrook et al., and translated by Peitang Huang et al., 3rd Edition, Science Press) or according to the product manual. Reagents or instruments used are commercially available conventional products if the manufacturers thereof are not specified. For example, MDA-MB-231 cells and U87-MG cells can be purchased from ATCC.

In the following examples of the present invention, BALB/c mice used were purchased from Guangdong Medical Experimental Animal Center.

In the following examples of the present invention, the positive control antibody MEDI9447 (oleclumab) used was produced by Akeso Biopharma Co. Ltd., the sequence of which is as same as the antibody SEQ ID NOs: 21-24 described in the Medlmmune Limited published patent with the publication number: US20160129108Al.

In the following examples of the present invention, AD2 used was purchased from Biolegend (Cat. No. 344002).

### Example 1. Preparation of Anti-CD73 Antibody 19F3

### 1. Preparation of hybridoma cell line LT014

The antigen used to prepare the anti-CD73 antibody was human NT5E-His (for NT5E, GenbankID: NP_002517.1, position: 1-552). Spleen cells of immunized mice were taken to fuse with myeloma cells of the mice to prepare hybridoma cells. Hybridoma cells were screened by indirect ELISA using human NT5E-Biotin (for NT5E, GenbankID: NP_002517.1, position: 1-552) as an antigen, and hybridoma cells capable of secreting an antibody specifically binding to CD73 were obtained. The hybridoma cells obtained by screening were subjected to limiting dilution to obtain a stable hybridoma cell line. The hybridoma cell line was named as hybridoma cell line LT014, and the monoclonal antibody secreted therefrom was named as 19F3.

The hybridoma cell line LT014 (also called CD73-19F3) was deposited at China Center for Type Culture Collection (CCTCC) on June 21, 2018 with a collection number of CCTCC NO: C2018137 and a collection address of Wuhan University, Wuhan, China, postal code: 430072.

### 2. Preparation of anti-CD73 antibody 19F3

The LT014 cell line prepared above was cultured with a chemical defined medium (CD medium, containing 1% Penicillin-Streptomycin) in a 5% CO₂ cell incubator at 37 °C. After 7 days, the cell culture supernatant was collected, subjected to high-speed centrifugation and vacuum filtration through a microfiltration membrane, and purified by using a HiTrap protein A HP column to obtain an antibody 19F3.

### Example 2. Sequence Analysis of Anti-CD73 Antibody 19F3

mRNA was extracted from the cell line LT014 cultured in Example 1 according to the method described in the manual of RNAprep pure Cell/Bacteria Kit (Tiangen, Cat. No. DP430).

cDNA was synthesized according to the manual of Invitrogen SuperScript^{®} III First-Strand Synthesis System for RT-PCR and amplified by PCR.

The PCR-amplified products were directly subjected to TA cloning according to the manual of the pEASY-T1 Cloning Kit (Transgen CT101).

The product obtained by performing TA cloning of the antibody 19F3 against CD73 on the LT014 cell line was directly sequenced. The sequencing results are as follows.

The nucleotide sequence of the heavy chain variable region is set forth in SEQ ID NO: 1 with a length of 363 bp.

The encoded amino acid sequence is set forth in SEQ ID NO: 2 with a length of 121 aa;
wherein the sequence of the heavy chain CDR1 is set forth in SEQ ID NO: 15, the sequence of the heavy chain CDR2 is set forth in SEQ ID NO: 16, and the sequence of the heavy chain CDR3 is set forth in SEQ ID NO: 17.

The nucleotide sequence of the light chain variable region is set forth in SEQ ID NO: 3 with a length of 339 bp.

The encoded amino acid sequence is set forth in SEQ ID NO: 4 with a length of 113 aa;
wherein the sequence of the light chain CDR1 is set forth in SEQ ID NO: 18, the sequence of the light chain CDR2 is set forth in SEQ ID NO: 19, and the sequence of the light chain CDR3 is set forth in SEQ ID NO: 20.

### Example 3. Design and Preparation of Light and Heavy Chains of Humanized Anti-Human CD73 Antibodies

### 1. Design of light and heavy chains of humanized anti-human CD73 antibodies 19F3H1L1, 19F3H2L2 and 19F3H2L3

Based on the three-dimensional crystal structure of human CD73 protein (Hage T, Reinemer P, Sebald W., Crystals of a 1:1 Complex Between Human Interleukin-4 and the Extracellular Domain of Its Receptor Alpha Chain, Eur. J. Biochem., 1998; 258(2):831-6.) and the sequence of antibody 19F3 obtained in Example 2, the variable region sequences of antibodies 19F3H1L1, 19F3H2L2 and 19F3H2L3 were obtained by computer modeling and mutation design (antibody constant region sequences from NCBI database: the heavy chain constant region is Ig gamma-1 chain C region, ACCESSION: P01857; the light chain constant region is Ig kappa chain C region, ACCESSION: P01834).

The designed variable region sequences are as follows:
(1) Sequences of heavy and light chain variable regions of humanized monoclonal antibody 19F3H1L1

The nucleotide sequence of the heavy chain variable region is set forth in SEQ ID NO: 5 with a length of 363 bp.

The encoded amino acid sequence is set forth in SEQ ID NO: 6 with a length of 121 aa, wherein the sequence of the heavy chain CDR1 is set forth in SEQ ID NO: 15, the sequence of the heavy chain CDR2 is set forth in SEQ ID NO: 16, and the sequence of the heavy chain CDR3 is set forth in SEQ ID NO: 17.

The nucleotide sequence of the light chain variable region is set forth in SEQ ID NO: 7 with a length of 339 bp.

The encoded amino acid sequence is set forth in SEQ ID NO: 8 with a length of 113 aa, wherein the sequence of the light chain CDR1 is set forth in SEQ ID NO: 18, the sequence of the light chain CDR2 is set forth in SEQ ID NO: 19, and the sequence of the light chain CDR3 is set forth in SEQ ID NO: 20.

### (2) Sequences of heavy and light chain variable regions of humanized monoclonal antibody 19F3H2L2

The nucleotide sequence of the heavy chain variable region is set forth in SEQ ID NO: 9 with a length of 363 bp.

The encoded amino acid sequence is set forth in SEQ ID NO: 10 with a length of 121 aa, wherein the sequence of the heavy chain CDR1 is set forth in SEQ ID NO: 15, the sequence of the heavy chain CDR2 is set forth in SEQ ID NO: 16, and the sequence of the heavy chain CDR3 is set forth in SEQ ID NO: 17.

The nucleotide sequence of the light chain variable region is set forth in SEQ ID NO: 11 with a length of 339 bp.

The encoded amino acid sequence is set forth in SEQ ID NO: 12 with a length of 113 aa, wherein the sequence of the light chain CDR1 is set forth in SEQ ID NO: 18, the sequence of the light chain CDR2 is set forth in SEQ ID NO: 19, and the sequence of the light chain CDR3 is set forth in SEQ ID NO: 20.

### (3) Sequences of heavy and light chain variable regions of humanized monoclonal antibody 19F3H2L3

The nucleotide sequence of the heavy chain variable region is set forth in SEQ ID NO: 9 with a length of 363 bp.

The encoded amino acid sequence is set forth in SEQ ID NO: 10 with a length of 121 aa, wherein the sequence of the heavy chain CDR1 is set forth in SEQ ID NO: 15, the sequence of the heavy chain CDR2 is set forth in SEQ ID NO: 16, and the sequence of the heavy chain CDR3 is set forth in SEQ ID NO: 17.

The nucleotide sequence of the light chain variable region is set forth in SEQ ID NO: 13 with a length of 339 bp.

The encoded amino acid sequence is set forth in SEQ ID NO: 14 with a length of 113 aa, wherein the sequence of the light chain CDR1 is set forth in SEQ ID NO: 18, the sequence of the light chain CDR2 is set forth in SEQ ID NO: 19, and the sequence of the light chain CDR3 is set forth in SEQ ID NO: 20.

### 2. Preparation of humanized antibodies 19F3H1L1, 19F3H2L2 and 19F3H2L3

The heavy chain constant regions were all Ig gamma-1 chain C region, ACCESSION: P01857; and the light chain constant regions were all Ig kappa chain C region, ACCESSION: P01834.

The heavy chain cDNA and light chain cDNA of 19F3H1L1, the heavy chain cDNA and light chain cDNA of 19F3H2L2 and the heavy chain cDNA and light chain cDNA of 19F3H2L3 were respectively cloned into pUC57simple (provided by GenScript) vectors to obtain pUC57simple-19F3H1, pUC57simple-19F3L1, pUC57simple-19F3H2, pUC57simple-19F3L2 and pUC57simple-19F3L3, respectively. Referring to the standard technique introduced in Molecular Cloning Laboratory Manual (Second Editi*on)*, the heavy and light chain full-length genes synthesized by EcoRI&HindIII digestion were subcloned into an expression vector pcDNA3.1 by an restriction enzyme (EcoRI&HindIII) through digestion to obtain expression plasmids pcDNA3.1-19F3H1, pcDNA3.1-19F3L1, pcDNA3.1-19F3H2, pcDNA3.1-19F3L2 and pcDNA3.1-19F3L3, and the heavy/light chain genes of the recombinant expression plasmids were further subjected to sequencing analysis. Then the designed gene combinations comprising corresponding light and heavy chain recombinant plasmids (pcDNA3.1-19F3H1/pcDNA3.1-19F3L1, pcDNA3.1-19F3H2/ pcDNA3.1-19F3L2 and pcDNA3.1-19F3H2/pcDNA3.1-19F3L3) were respectively co-transfected into 293F cells, and the culture solutions were collected and purified. After the sequences were verified, endotoxin-free expression plasmids were prepared, and were transiently transfected into HEK293 cells for antibody expression. The culture solutions were collected after 7 days, and subjected to affinity purification on a Protein A column to obtain humanized antibodies.

### 3. Preparation of humanized antibodies 19F3H1L1(hG1DM), 19F3H2L2(hG1DM) and 19F3H2L3(hG1DM)

The light chain constant regions of the antibodies 19F3H1L1(hG1DM), 19F3H2L2(hG1DM) and 19F3H2L3(hG1DM) are the Ig kappa chain C region, ACCESSION: P01834, see SEQ ID NO: 22.

On the basis of Ig gamma-1 chain C region, ACCESSION: P01857, humanized antibodies were obtained by introducing a leucine-to-alanine point mutation at position 234 (L234A) and a leucine-to-alanine point mutation at position 235 (L235A) in the heavy chain constant region, see SEQ ID NO: 21, and were designated as 19F3H1L1(hG1DM), 19F3H2L2(hG1DM) and 19F3H2L3(hG1DM), respectively.

The heavy chain cDNA and light chain cDNA of 19F3H1L1(hG1DM), the heavy chain cDNA and light chain cDNA of 19F3H2L2(hG1DM) and the heavy chain cDNA and light chain cDNA of 19F3H2L3(hG1DM) were respectively cloned into pUC57simple (provided by Genscript) vectors to obtain pUC57simple-19F3H1(hG1DM), pUC57simple-19F3L1, pUC57simple-19F3H2(hG1DM), pUC57simple-19F3L2 and pUC57simple-19F3L3, respectively. Referring to the standard techniques described in Molecular Cloning: A Laboratory Manual (Second Editi*on)*, the heavy and light chain full-length genes synthesized by EcoRI&HindIII digestion were subcloned into an expression vector pcDNA3.1 by an restriction enzyme (EcoRI&HindIII) through digestion to obtain expression plasmids pcDNA3.1-19F3H1(hG1DM), pcDNA3.1-19F3L1, pcDNA3.1-19F3H2(hG1DM),pcDNA3.1-19F3L2 an pcDNA3.1-19F3L3, and the heavy/light chain genes of the recombinant expression plasmids were further subjected to sequencing analysis. Then the designed gene combinations comprising corresponding light and heavy chain recombinant plasmids (pcDNA3.1-19F3H1(hG1DM)/pcDNA3.1-19F3L1, pcDNA3.1-19F3H2(hG1DM)/ pcDNA3.1-19F3L2 and pcDNA3.1-19F3H2(hG1DM)/pcDNA3.1-19F3L3) were respectively co-transfected into 293F cells, and the culture solutions were collected and purified. After the sequences were verified, endotoxin-free expression plasmids were prepared, and were transiently transfected into HEK293 cells for antibody expression. The culture solutions were collected after 7 days, and subjected to affinity purification on a Protein A column to obtain humanized antibodies.

### Example 4. Determination of Binding Activity of Anti-CD73 Antibody to Antigen Human NT5E-Biotin by ELISA

Experimental steps: a microplate was coated with streptavidin at 2 µg/mL, and then the microplate was incubated at 4 °C for 12 hours. The antigen-coated microplate was rinsed for once with PBST after incubation, and then blocked with a PBST solution containing 1% BSA as a microplate blocking solution for 2 hours. After blocking, the microplate was washed 3 times with PBST. Then, 0.5 µg/mL antigen human NT5E-Biotin was added, and the plate was washed 3 times with PBST after incubated at 37 °C for 30 minutes. The antibody serially diluted with the PBST solution was added to the wells of the microplate. The antibody dilution gradients are shown in Table 1. The microplate containing the test antibodies was incubated at 37 °C for 30 minutes, and then washed 3 times with PBST. After the plate was washed, the secondary antibody working solution of HRP-labeled goat anti-human IgG (H+L) (Jackson, Cat. No. 109-035-088) diluted in a ratio of 1:5000 or HRP-labeled goat anti-mouse IgG (H+L) (Jackson, Cat. No. 115-035-062) diluted in a ratio of 1:5000 was added, and then the plate was incubated at 37 °C for 30 minutes. After incubation, the plate was washed 4 times with PBST, TMB (Neogen, 308177) was added in the dark for color development for 5 min, and then a stop solution was added to terminate chromogenic reaction. The microplate was put into a microplate reader immediately, and the OD value of each well in the microplate was read at the light wavelength of 450 nm. The data were analyzed by SoftMax Pro 6.2.1.

OD values of doses for detecting the binding of anti-CD73 antibody to the antigen human NT5E-Biotin are shown in Table 1. The binding EC₅₀ of the antibody was calculated by curve fitting using antibody concentration as the abscissa and absorbance value as the ordinate, and the results are shown in Table 1 below.

Experimental results show that the antibodies 19F3 H1L1, 19F3 H2L2 and 19F3 H2L3 and the murine antibody 19F3 can effectively bind to human NT5E-Biotin, and the binding efficiency is in a dose-dependent relationship. Under the basically same experimental conditions, EC₅₀ of 19F3 H1L1 binding to human NT5E-Biotin was 0.049 nM, EC₅₀ of 19F3 H2L2 binding to human NT5E-Biotin was 0.064 nM, EC₅₀ of 19F3 H2L3 binding to human NT5E-Biotin was 0.061 nM, EC₅₀ of the positive drug MEDI9447 for the same target binding to human NT5E-Biotin was 0.048 nM, and EC₅₀ of murine antibody 19F3 binding to human NT5E-Biotin was 0.018 nM.

The above experimental results show that the binding activity of 19F3 H1L1, 19F3 H2L2, 19F3 H2L3 and murine antibody 19F3 to human NT5E-Biotin respectively is comparable to that of the positive drug MEDI9447 for the same target under the same experimental conditions, indicating that 19F3 H1L1, 19F3 H2L2 and 19F3 H2L3 have the function of effectively binding to CD73.

**Table 1. Detection results of binding activity of 19F3H1L1, 19F3H2L2, 19F3H2L3 and murine antibody 19F3 to HNT5E-Biotin**

| **Antibody dilution (µg/mL)** | **Antigen coating: SA (2 µg/mL)** | | | | | | | | | |
|---|---|---|---|---|---|---|---|---|---|---|
| | **Human NT5E-Biotin (0.5 µg/mL)** | | | | | | | | | |
| | **19F3 H1L1** | | **19F3 H2L2** | | **19F3 H2L3** | | **MEDI9447** | | **19F3** | |
| 0.333 | 2.648 | 2.640 | 2.598 | 2.688 | 2.623 | 2.588 | 2.548 | 2.527 | 2.706 | 2.743 |
| 1:3 | 2.601 | 2.697 | 2.578 | 2.618 | 2.581 | 2.582 | 2.573 | 2.604 | 2.736 | 2.763 |
| 1:9 | 2.407 | 2.332 | 2.163 | 2.330 | 2.186 | 2.257 | 2.268 | 2.284 | 2.566 | 2.641 |
| 1:27 | 1.821 | 1.820 | 1.579 | 1.680 | 1.626 | 1.649 | 1.774 | 1.742 | 2.330 | 2.361 |
| 1:81 | 1.044 | 1.035 | 0.870 | 0.933 | 0.918 | 0.931 | 1.058 | 1.030 | 1.693 | 1.769 |
| 1:243 | 0.525 | 0.516 | 0.434 | 0.454 | 0.450 | 0.457 | 0.536 | 0.528 | 1.001 | 1.000 |
| 1:729 | 0.260 | 0.273 | 0.239 | 0.247 | 0.241 | 0.252 | 0.272 | 0.275 | 0.446 | 0.470 |
| 0 | 0.125 | 0.123 | 0.119 | 0.123 | 0.120 | 0.123 | 0.121 | 0.116 | 0.060 | 0.062 |
| Secondary antibody | HRP-labeled goat anti-human IgG (H+L) (1:5000) | | | | | | | | HRP-labeled goat anti-mouse IgG (H+L) (1:5000) | |
| EC₅₀ (nM) | 0.049 | | 0.064 | | 0.061 | | 0.048 | | 0.018 | |

### Example 5. Kinetic Parameters for Binding of Humanized Antibodies 19F3H2L3, 19F3H2L2 and MEDI9447 to the Antigen Human HNT5E (1-552)-His Determined using Fortebio Molecular Interaction Instrument

The sample dilution buffer was PBST, pH 7.4. 5 µg/mL antibody was immobilized at on the Protein A sensor for 15 s. The sensor was equilibrated in the buffer for 120 s. The binding of the immobilized antibody on the sensor to the antigen human NT5E(1-552)-his at concentrations of 3.125-200 nM (two-fold dilution) was determined for 120 s. The antigen-antibody was dissociated in the buffer for 600 s. The sensor was refreshed with 10 mM glycine solution (pH 1.5). The detection temperature was 37 °C, the detection frequency was 0.6 Hz, and the sample plate shaking rate was 1000 rpm. The data were analyzed by 1:1 model fitting to obtain affinity constants.

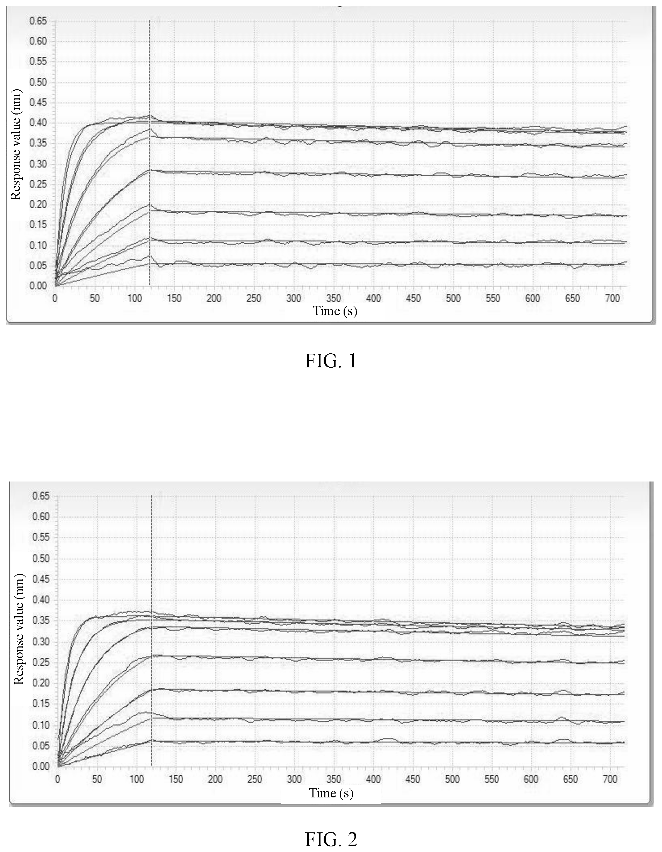

The results of measuring the affinity constants of humanized antibodies 19F3H2L3, 19F3H2L2 and MEDI9447 (as control antibodies) and human CD73 are shown in Table 2, and the detection results are shown in FIGs. 1-3.

As shown in Table 2 and FIGs. 1-3, the affinity constants of humanized antibodies 19F3H2L3, 19F3H2L2 and MEDI9447 and human CD73 were 1.04E-10 M, 2.36E-10 M, 2.59E-10 M and 1.04E-10 M sequentially.

The above experimental results show that the binding ability of 19F3H2L3 and 19F3H2L2 to HNT5E(1-552)-his is comparable, indicating that the humanized antibodies 19F3H2L3 and 19F3H2L2 have strong binding capacity to human CD73.

**Table 2. Determination of affinity constants of 19F3H2L3, 19F3H2L2 and HNT5E(1-552)-his**

| **Test antibodies** | **KD (M)** | **kon(1/Ms)** | **S E (kon)** | **kdis(1/s)** | **S E(kdis)** | **Rmax (nm)** |
|---|---|---|---|---|---|---|
| 19F3 H2L3 | 2.36E-10 | 4.82E+05 | 7.74E+03 | 1.14E-04 | 5.87E-06 | 0.34-0.41 |
| 19F3 H2L2 | 2.59E-10 | 4.80E+05 | 6.44E+03 | 1.24E-04 | 4.86E-06 | 0.35-0.39 |
| MEDI9447 | 1.04E-10 | 2.34E+05 | 3.20E+03 | 2.44E-05 | 5.02E-06 | 0.59-0.82 |

| | | | | | | |
|---|---|---|---|---|---|---|
| K_{D} is the affinity constant; K_{D} = kdis/kon | | | | | | |

### Example 6. Binding Ability of 19F3H2L3 Antibody Determined by FACS

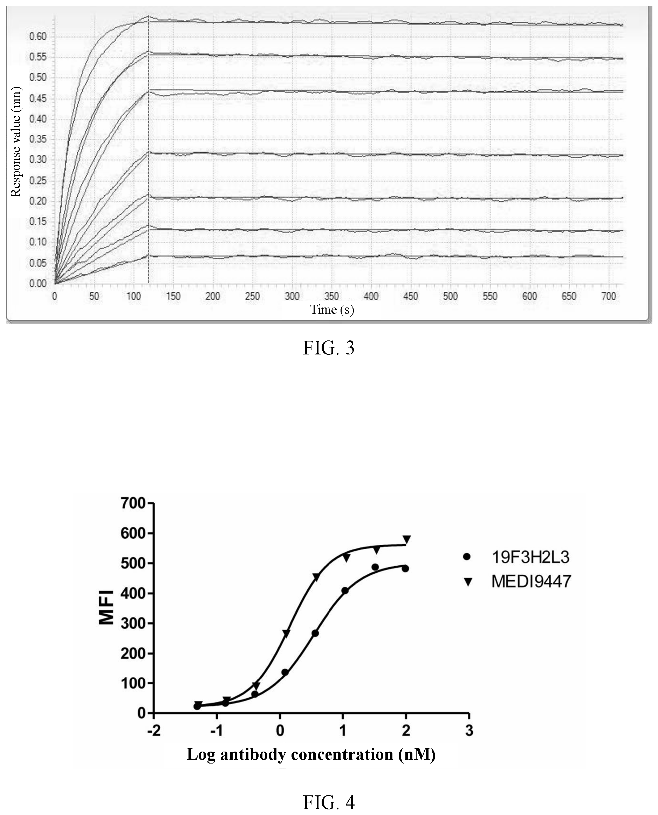

MDA-MB-231 cells in logarithmic phase were digested with conventional trypsin, centrifuged, and resuspended in PBSA. The cells were filled into 1.5 mL centrifuge tubes (0.3 million cells in each tube) and centrifuged at 5600 rpm for 5 min to remove the supernatant. 100 µL of CD73 antibodies diluted by PBSA with the final concentrations of 100 nM, 33.33 nM, 11.11 nM, 3.7 nM, 1.23 nM, 0.41 nM, 0.14 nM and 0.05 nM were added, respectively. The system was mixed gently and uniformly, and then was incubated on ice for 1 h. Then 500 µL of PBSA was added, and the mixture was centrifuged at 5600 rpm for 5 min to remove the supernatant. The 500-fold diluted FITC labeled goat anti-human IgG secondary antibody (Jackson, Cat. No. 109-095-098) was added to resuspend cell precipitates, and the mixture was incubated on ice in the dark for 0.5 h. 500 µL of PBSA was added, and the mixture was centrifuged at 5600 rpm for 5 min to remove the supernatant. At last, 200 µL of PBSA was added to resuspend cell precipitates, and the mixture was transferred to a flow tube for detection by a flow cytometer (BD FACSCalibur^{™}). Results are shown in Table 3 and FIG. 4, the binding ability of 19F3H2L3 to CD73 endogenously expressed in MDA-MB-231 is comparable to that of the positive drug MEDI9447 for the same target, and the binding is in a dose-dependent relationship. Under the same experimental conditions, EC₅₀ of 19F3H2L3 binding to CD73 endogenously expressed in MDA-MB-231 is 3.423 nM, and EC₅₀ of MEDI9447 binding to CD73 endogenously expressed in MDA-MB-231 is 1.433 nM.

The above experimental results show that the binding activity of 19F3H2L3 to CD73 endogenously expressed in MDA-MB-231 is comparable to that of the positive drug MEDI9447 for the same target under the same experimental conditions, indicating that 19F3H2L3 has the function of effectively binding to CD73.

**Table 3. Binding ability of 19F3H2L3 to CD73 on the surface of MDA-MB-231 cells determined by FACS**

| **Absorbance (nM)** | **0.05** | **0.14** | **0.41** | **1.23** | **3.70** | **11.11** | **33.33** | **100.00** | **EC₅₀** |
|---|---|---|---|---|---|---|---|---|---|
| **MEDI9447** | 28.38 | 43.18 | 90.96 | 266.72 | 454.45 | 518.94 | 545.63 | 580.76 | **1.433** |
| **19F3H2L3** | 20.04 | 31.49 | 61.09 | 135.31 | 264.86 | 407.09 | 485.32 | 480.42 | **3.423** |

### Example 7. Determination of Inhibitory Activity of Anti-CD73 Antibody on Enzyme Activity of CD73 Endogenously Expressed in Cells

### 1. Detection of inhibitory activity of anti-CD73 antibody on enzyme activity of CD73 endogenously expressed in MDA-MB-231 cells

The experimental procedures were as follows. MDA-MB-231 cells in logarithmic phase in good condition were taken, resuspended in a serum-free RPMI-1640 culture solution, and then counted. The MDA-MB-231 cells were seeded into a 96-well plate at 3×10⁴ cells/100 µL/well. The antibody was diluted with the serum-free RPMI-1640 culture solution at an initial concentration of 200 µg/mL (serial 2.5-fold dilution). The antibody was added to the 96-well plate at 50 µL/well, and the plate was incubated at 37 °C for 1 hour. After 1 hour, 50 µL of RPMI-1640-diluted 600 µM AMP was added to each well. After 3 hours, 25 µL of cell culture supernatant was taken and transferred to a new 96-well plate, and 25 µL of 100 µM ATP was added to each well. 50 µL of CTG (CellTiter-Glo^{®} One Solution Assay, promega, Cat. No. G8461) color developing solution was added to each well for color development, and data were read by a multi-label microplate tester (PerkinElmer 2140-0020).

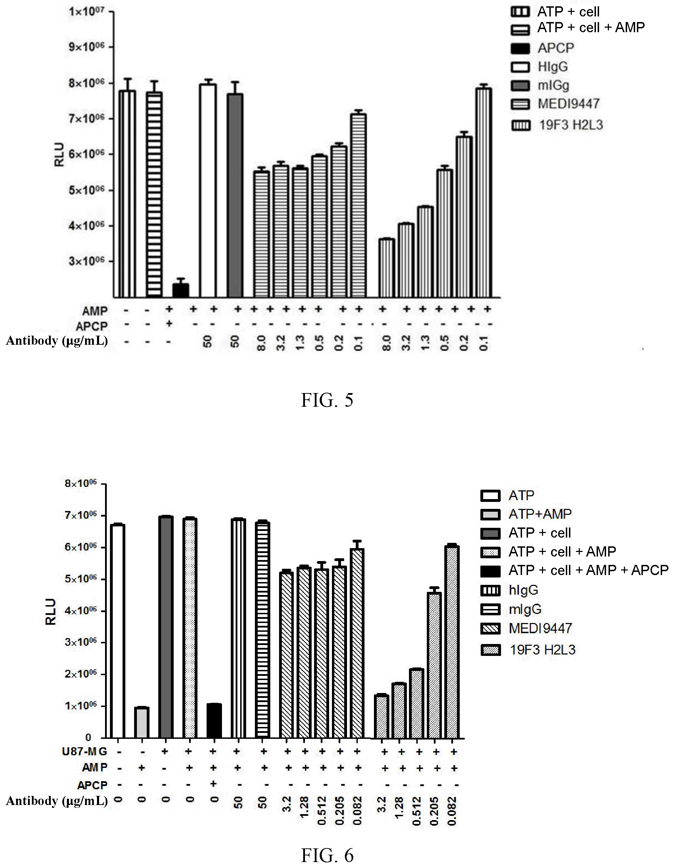

Experimental results: as shown in FIG. 5, both 19F3H2L3 and the positive control drug MEDI9447 for the same target showed dose-dependent inhibition of the activity of CD73 endogenously expressed in MDA-MB-231 converting AMP into adenosine A through enzyme catalysis, thereby reducing the produced mean fluorescence intensity RLU produced in a dose-dependent manner.

The above experimental results show that the added AMP can be subjected to enzyme catalysis of CD73 endogenously expressed on the cell surface by MDA-MB-231 and then converted into adenosine A under the condition of no CD73 antibody treatment, so that the inhibition of luciferase activity is relieved. However, after addition of the antibody, as CD73 was bound by the antibody, its enzymatic activity was reduced, so that AMP could not be converted into adenosine. It is suggested that the anti-CD73 antibody effectively inhibits the enzyme activity reaction of CD73 in a non-substrate competition mode and reduces the production of adenosine.

### 2. Determination of enzyme activity by addition of anti-CD73 antibody to U87-MG cells

The experimental procedures were as follows. U87-MG cells in logarithmic phase in good condition were taken, resuspended in a serum-free RPMI-1640 culture solution, and then counted. The U87-MG cells were seeded into a 96-well plate at 3×10⁴ cells/100 µL/well. The antibody was diluted with the serum-free RPMI-1640 culture solution at an initial concentration of 200 µg/mL (serial 2.5-fold dilution). The antibody was added to the 96-well plate at 50 µL/well, and the plate was incubated at 37 °C for 1 hour. After 1 hour, 50 µL of RPMI-1640-diluted 600 µM AMP was added to each well. After 3 hours, 25 µL of cell culture supernatant was taken and transferred to a new 96-well plate, and 25 µL of 100 µM ATP was added to each well. 50 µL of CTG (CellTiter-Glo^{®} One Solution Assay, promega, Cat. No. G8461) color developing solution was added to each well for color development, and data were read by a multi-label microplate tester (PerkinElmer 2140-0020).

Experimental results: as shown in FIG. 6, both 19F3H2L3 and the positive control drug MEDI9447 for the same target showed dose-dependent inhibition of the activity of CD73 endogenously expressed in MDA-MB-231 converting AMP into adenosine through enzyme catalysis, thereby reducing the produced mean fluorescence intensity RLU produced in a dose-dependent manner.

The above experimental results show that the added AMP can be subjected to enzyme catalysis of CD73 endogenously expressed on the cell surface by U87-MG and then converted into adenosine under the condition of no CD73 antibody treatment, so that the inhibition of luciferase activity is relieved. However, after addition of the antibody, as CD73 was bound by the antibody, its enzymatic activity was reduced, so that AMP could not be converted into adenosine. It is suggested that the anti-CD73 antibody effectively inhibits the enzyme activity reaction of CD73 in a non-substrate competition mode and reduces the production of adenosine.

### Example 8. Dynamic Affinity Determination of Anti-CD73 Antibodies and FcγRIIIa Mediating Antibody-Dependent Cytotoxic Effects

The sample dilution buffer was PBS (0.02% Tween-20, 0.1% BSA, pH 7.4). 0.5 µg/mL FcγRIIIa (from Sino Biological, also known as CD16a) was immobilized on the SA sensor for 120 s. The sensor was equilibrated in a buffer for 60 s, and the binding of the immobilized CD16a on the sensor to the antibodies at concentrations of 31.3-500 nM (serial two-fold dilution) was determined for 60 s. The antibody-antigen was dissociated in buffer for 60 s. The sensor was refreshed with 10 mM NaOH. The detection temperature was 30 °C and the frequency was 0.6 Hz. The data were analyzed by 1:1 model fitting to obtain affinity constants.

As shown in Table 4, 19F3H2L3(hG1DM) did not bind to FcγRIIIa, whereas MEDI9447 had binding activity to FcγRIIIa, indicating that 19F3H2L3(hG1DM) had a lower risk of causing antibody-dependent cytotoxic effects in CD73-expressing cells, whereas MEDI9447 had a risk of causing antibody-dependent cytotoxic effects.

CD73 is abundantly expressed in vascular endothelium and normal tissues, and binds to FcγRIIIa to mediate antibody-dependent cytotoxic effects, resulting in damage to vascular endothelial cells and normal tissues, which has significant effect on the safety of antibody drugs.

**Table 4. Dynamic affinity determination of anti-CD73 antibodies and FcγRIIIa mediating antibody-dependent cytotoxic effects**

| | **KD (M)** | **kon (1/Ms)** | **Standard error (kon)** | **Kdis (1/s)** | **Standard error (kdis)** |
|---|---|---|---|---|---|
| 19F3H2L3(hG1DM) | N/A | N/A | N/A | N/A | N/A |
| MEDI9447 | 1.41E-07 | 2.43E+05 | 4.36E+04 | 3.42E-02 | 2.08E-03 |
| Wild-type IgG1 antibody | 1.25E-07 | 1.76E+05 | 2.22E+04 | 2.20E-02 | 1.11E-03 |

| | | | | | |
|---|---|---|---|---|---|
| KD = kdis/kon | | | | | |

### SEQUENCE LISTING

The nucleotide sequence of the heavy chain variable region of 19F3 with CDR sequences underlined: (SEQ ID NO: 1)

GAGGTGCAGCTGCAGCAGTCCGGACCAGAGCTGGTGAAGCCTGGCGCCTCCATGCGG
ATGTCTTGTAAGGCCTCTGGCTACAGCTTCACCGGCTATACAATGAACTGGGTGAAG
CAGTCTCACGGCAAGAATCTGGAGTGGATCGGCCTGATCAACCCTTACAATGCCGGC
ACCAGCTATAACCAGAAGTTTAAGGGCAAGGCCACCCTGACAGTGGACAAGAGCTCC
TCTACCGCCTACATGGAGCTGCTGTCCCTGACATCTGAGGATAGCGCCGTGTACTATT
GCGCCCGGTCCGAGTACAGATATGGCGGCGACTACTTTGATTATTGGGGCCAGGGCA
CCACACTGACAGTGAGCTCC

The amino acid sequence of the heavy chain variable region of 19F3 with CDR sequences underlined: (SEQ ID NO: 2)

EVQLQQSGPELVKPGASMRMSCKASGYSFTGYTMNWVKQSHGKNLEWIGLINPYNAGT
SYNQKFKGKATLTVDKSSSTAYMELLSLTSEDSAVYYCARSEYRYGGDYFDYWGQGTT
LTVSS

The nucleotide sequence of the light chain variable region of 19F3 with CDR sequences underlined: (SEQ ID NO: 3)

GACATCGTGATGACCCAGTCTCCAAGCTCCCTGGCAATGAGCGTGGGACAGAAGGTG
ACAATGTCTTGTAAGTCTAGCCAGAGCCTGCTGAACTCCTCTAATCAGAAGAACTAC
CTGGCCTGGTATCAGCAGAAGCCAGGCCAGTCTCCCAAGCTGCTGGTGTACTTTGCC
AGCACCAGGGAGTCCGGAGTGCCTGACAGATTCATCGGCTCCGGCTCTGGCACAGAC
TTCACCCTGACAATCAGCTCCGTGCAGGCAGAGGACCTGGCAGATTATTTCTGCCAG
CAGCACTACGACACCCCTTATACATTTGGCGGCGGCACCAAGCTGGAGATCAAG

The amino acid sequence of the light chain variable region of 19F3 with CDR sequences underlined: (SEQ ID NO: 4)

DIVMTQSPSSLAMSVGQKVTMSCKSSQSLLNSSNQKNYLAWYQQKPGQSPKLLVYFAST
RESGVPDRFIGSGSGTDFTLTISSVQAEDLADYFCQQHYDTPYTFGGGTKLEIK

The nucleotide sequence of the heavy chain variable region of 19F3H1L1 with CDR sequences underlined: (SEQ ID NO: 5)

CAGGTGCAGCTGCAGCAGTCTGGAGCAGAGGTGGTGAAGCCAGGAGCCTCTATGAA
GATGAGCTGTAAGGCCAGCGGCTACTCCTTCACCGGCTATACAATGAACTGGGTGAA
GCAGGCCCACGGCCAGAATCTGGAGTGGATCGGCCTGATCAACCCTTACAATGCCGG
CACCTCTTATAACCAGAAGTTTCAGGGCAAGGCCACCCTGACAGTGGACAAGTCCAC
CTCTACAGCCTACATGGAGCTGAGCTCCCTGCGGAGCGAGGATACAGCCGTGTACTA
TTGCGCCCGGTCCGAGTACAGATATGGCGGCGACTACTTTGATTATTGGGGCCAGGG
CACCACACTGACCGTGTCTAGC

The amino acid sequence of the heavy chain variable region of 19F3H1L1 with CDR sequences underlined: (SEQ ID NO: 6)

QVQLQQSGAEVVKPGASMKMSCKASGYSFTGYTMNWVKQAHGQNLEWIGLINPYNAG
TSYNQKFQGKATLTVDKSTSTAYMELSSLRSEDTAVYYCARSEYRYGGDYFDYWGQGT
TLTVSS

The nucleotide sequence of the light chain variable region of 19F3H1L1 with CDR sequences underlined: (SEQ ID NO: 7)

GACATCGTGATGACCCAGTCCCCAAGCTCCCTGGCAATGTCTGTGGGAGAGAGGGTG
ACAATGTCCTGTAAGTCTAGCCAGTCTCTGCTGAACTCCTCTAATCAGAAGAACTACC
TGGCCTGGTATCAGCAGAAGCCCGGCCAGGCCCCTAAGCTGCTGGTGTACTTTGCCTC
TACCAGGGAGAGCGGAGTGCCAGACAGATTCTCTGGCAGCGGCTCCGGCACAGACTT
CACCCTGACAATCAGCTCCGTGCAGGCAGAGGACCTGGCAGATTATTTCTGCCAGCA
GCACTACGATACCCCCTATACATTTGGCGGCGGCACCAAGCTGGAGATCAAG

The amino acid sequence of the light chain variable region of 19F3H1L1 with CDR sequences underlined: (SEQ ID NO: 8)

DIVMTQSPSSLAMSVGERVTMSCKSSQSLLNSSNQKNYLAWYQQKPGQAPKLLVYFAST
RESGVPDRFSGSGSGTDFTLTISSVQAEDLADYFCQQHYDTPYTFGGGTKLEIK

The nucleotide sequence of the heavy chain variable regions of 19F3H2L2 and 19F3H2L3 with CDR sequences underlined: (SEQ ID NO: 9)

CAGGTGCAGCTGGTGCAGTCTGGAGCAGAGGTGGTGAAGCCAGGAGCCTCTGTGAA

GGTGAGCTGTAAGGCCAGCGGCTACTCCTTCACCGGCTATACAATGAACTGGGTGAG
GCAGGCACCAGGACAGAATCTGGAGTGGATCGGCCTGATCAACCCTTACAATGCCGG
CACCTCTTATAACCAGAAGTTTCAGGGCAAGGTGACCCTGACAGTGGACAAGTCCAC
CTCTACAGCCTACATGGAGCTGAGCTCCCTGCGGAGCGAGGATACAGCCGTGTACTA
TTGCGCCCGGTCCGAGTACAGATATGGCGGCGACTACTTTGATTATTGGGGCCAGGG
CACCACACTGACCGTGTCTAGC

The amino acid sequence of the heavy chain variable regions of 19F3H2L2 and 19F3H2L3 with CDR sequences underlined: (SEQ ID NO: 10)

QVQLVQSGAEVVKPGASVKVSCKASGYSFTGYTMNWVRQAPGQNLEWIGLINPYNAGT
SYNQKFQGKVTLTVDKSTSTAYMELSSLRSEDTAVYYCARSEYRYGGDYFDYWGQGTT
LTVSS

The nucleotide sequence of the light chain variable region of 19F3H2L2 with CDR sequences underlined: (SEQ ID NO: 11)

GACATCGTGATGACCCAGTCCCCAAGCTCCCTGGCCGTGTCTGTGGGAGAGCGGGTG
ACAATCTCCTGTAAGTCTAGCCAGTCTCTGCTGAACTCCTCTAATCAGAAGAACTACC
TGGCCTGGTATCAGCAGAAGCCCGGCCAGGCCCCTAAGCTGCTGATCTACTTCGCCTC
TACCAGGGAGAGCGGAGTGCCAGACAGATTCTCTGGCAGCGGCTCCGGCACAGACTT
CACCCTGACAATCAGCTCCGTGCAGGCAGAGGACGTGGCAGATTACTATTGCCAGCA
GCACTACGATACCCCCTATACATTTGGCGGCGGCACCAAGCTGGAGATCAAG

The amino acid sequence of the light chain variable region of 19F3H2L2 with CDR sequences underlined: (SEQ ID NO: 12)

DIVMTQSPSSLAVSVGERVTISCKSSQSLLNSSNQKNYLAWYQQKPGQAPKLLIYFASTRE
SGVPDRFSGSGSGTDFTLTISSVQAEDVADYYCQQHYDTPYTFGGGTKLEIK

The nucleotide sequence of the light chain variable region of 19F3H2L3: (SEQ ID NO: 13)

GACATCGTGATGACCCAGTCCCCAAGCTCCCTGGCCGTGTCTGTGGGAGAGCGGGTG
ACAATCTCCTGTAAGTCTAGCCAGTCTCTGCTGAACTCCTCTAATCAGAAGAACTACC
TGGCCTGGTATCAGCAGAAGCCCGGCCAGGCCCCTAAGCTGCTGATCTACTTCGCCTC
TACCAGGGAGAGCGGAGTGCCAGACAGATTCTCTGGCAGCGGCTCCGGCACAGACTT
CACCCTGACAATCAGCTCCCTGCAGGCAGAGGACGTGGCCGTGTACTATTGCCAGCA
GCACTACGATACCCCCTATACATTTGGCGGCGGCACCAAGCTGGAGATCAAG

The amino acid sequence of the light chain variable region of 19F3H2L3 with CDR sequences underlined: (SEQ ID NO: 14)

DIVMTQSPSSLAVSVGERVTISCKSSQSLLNSSNQKNYLAWYQQKPGQAPKLLIYFASTRE
SGVPDRFSGSGSGTDFTLTISSLQAEDVAVYYCQQHYDTPYTFGGGTKLEIK

The CDR regions of 19F3, 19F3H1L1, 19F3H2L2 and 19F3H2L3
HCDR1: GYSFTGYT (SEQ ID NO: 15)
HCDR2: INPYNAGT (SEQ ID NO: 16)
HCDR3: ARSEYRYGGDYFDY (SEQ ID NO: 17)
LCDR1: QSLLNSSNQKNY (SEQ ID NO: 18)
LCDR2: FAS (SEQ ID NO: 19)
LCDR3: QQHYDTPYT (SEQ ID NO: 20)

The sequence of the heavy chain constant regions (330 aa, mutation sites are underlined in bold) of 19F3H1L1(hG1DM), 19F3H2L2(hG1DM) and 19F3H2L3(hG1DM):

ASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKKVEPKSCDKTHTCPPCPAPEAAGG
PSVFLFPPKPKDTLMISRTPEVTCVVVDVSHEDPEVKFNWYVDGVEVHNAKTKPREEQY
NSTYRVVSVLTVLHQDWLNGKEYKCKVSNKALPAPIEKTISKAKGQPREPQVYTLPPSRD
ELTKNQVSLTCLVKGFYPSDIAVEWESNGQPENNYKTTPPVLDSDGSFFLYSKLTVDKSR
WQQGNVFSCSVMHEALHNHYTQKSLSLSPGK
(SEQ ID NO: 21)

The sequence of the light chain constant regions (107 aa) of 19F3H1L1(hG1DM), 19F3H2L2(hG1DM) and 19F3H2L3(hG1DM):


 (SEQ ID NO: 22)

Additional aspects and embodiments are set out in the following numbered paragraphs which form part of the description.
1. An anti-CD73 (for example, human CD73) antibody or an antigen-binding fragment thereof, wherein the anti-CD73 antibody comprises:
   HCDR1, HCDR2 and HCDR3 contained in a heavy chain variable region set forth in SEQ ID NO: 2, SEQ ID NO: 6 or SEQ ID NO: 10; and
   LCDR1, LCDR2 and LCDR3 contained in a light chain variable region set forth in SEQ ID NO: 4, SEQ ID NO: 8, SEQ ID NO: 12 or SEQ ID NO: 14;
   preferably, according to an IMGT numbering system,
   the anti-CD73 antibody comprises:
      HCDR1, comprising or consisting of an amino acid sequence set forth in SEQ ID NO: 15, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2 or 3) conservative amino acid mutations (preferably substitutions, insertions or deletions) compared to the sequence,
      HCDR2, comprising or consisting of an amino acid sequence set forth in SEQ ID NO: 16, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2 or 3) conservative amino acid mutations (preferably substitutions, insertions or deletions) compared to the sequence,
      HCDR3, comprising or consisting of an amino acid sequence set forth in SEQ ID NO: 17, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2 or 3) conservative amino acid mutations (preferably substitutions, insertions or deletions) compared to the sequence,
      LCDR1, comprising or consisting of an amino acid sequence set forth in SEQ ID NO: 18, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2 or 3) conservative amino acid mutations (preferably substitutions, insertions or deletions) compared to the sequence,
      LCDR2, comprising or consisting of an amino acid sequence set forth in SEQ ID NO: 19, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence or an amino acid, or a sequence having one or more (preferably 1, 2 or 3) conservative amino acid mutations (preferably substitutions, insertions or deletions) compared to the sequence, and
      LCDR3, comprising or consisting of an amino acid sequence set forth in SEQ ID NO: 20, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2 or 3) conservative amino acid mutations (preferably substitutions, insertions or deletions) compared to the sequence.
2. The anti-CD73 antibody or the antigen-binding fragment thereof according to paragraph 1, wherein the heavy chain variable region of the antibody comprises or consists of the following sequences:
   SEQ ID NO: 2, SEQ ID NO: 6 or SEQ ID NO: 10, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to SEQ ID NO: 2, SEQ ID NO: 6 or SEQ ID NO: 10, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 2, SEQ ID NO: 6 or SEQ ID NO: 10; and
   the light chain variable region of the antibody comprises or consists of the following sequences:
      SEQ ID NO: 4, SEQ ID NO: 8, SEQ ID NO: 12 or SEQ ID NO: 14, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to SEQ ID NO: 4, SEQ ID NO: 8, SEQ ID NO: 12 or SEQ ID NO: 14, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 4, SEQ ID NO: 8, SEQ ID NO: 12 or SEQ ID NO: 14.
3. The anti-CD73 antibody or the antigen-binding fragment thereof according to paragraph 1 or 2, wherein
   the heavy chain variable region of the antibody comprises an amino acid sequence set forth in SEQ ID NO: 2, and the light chain variable region of the antibody comprises an amino acid sequence set forth in SEQ ID NO: 4;
   the heavy chain variable region of the antibody comprises an amino acid sequence set forth in SEQ ID NO: 6, and the light chain variable region of the antibody comprises an amino acid sequence set forth in SEQ ID NO: 8;
   the heavy chain variable region of the antibody comprises an amino acid sequence set forth in SEQ ID NO: 10, and the light chain variable region of the antibody comprises an amino acid sequence set forth in SEQ ID NO: 12; or
   the heavy chain variable region of the antibody comprises an amino acid sequence set forth in SEQ ID NO: 10, and the light chain variable region of the antibody comprises an amino acid sequence set forth in SEQ ID NO: 14.
4. The anti-CD73 antibody or the antigen-binding fragment thereof according to paragraph 1 or 2, wherein the antibody comprises a heavy chain constant region having a sequence set forth in SEQ ID NO: 21, and a light chain constant region having a sequence set forth in SEQ ID NO: 22; or the heavy chain constant region is Ig gamma-1 chain C region, ACCESSION: P01857, and the light chain constant region is Ig kappa chain C region, ACCESSION: P01834).
5. The anti-CD73 antibody or the antigen-binding fragment thereof according to paragraph 1 or 2, wherein the antibody is a monoclonal antibody (preferably a heavy chain amino acid sequence set forth in SEQ ID NO: 35 and a light chain amino acid sequence set forth in SEQ ID NO: 36), a humanized antibody, a chimeric antibody and a multispecific antibody (e.g., a bispecific antibody).
6. The anti-CD73 antibody or the antigen-binding fragment thereof according to paragraph 1 or 2, wherein the antigen-binding fragment is selected from Fab, Fab', F(ab')₂, Fd, Fv, dAb, Fab/c, a complementarity determining region fragment, a single chain antibody (e.g., scFv), a humanized antibody, a chimeric antibody and a bispecific antibody.
7. An isolated polypeptide, selected from the group consisting of:
   (1) an isolated polypeptide, comprising sequences set forth in SEQ ID NO: 15, SEQ ID NO: 16 and SEQ ID NO: 17, wherein the polypeptide specifically binds to CD73 as a part of an anti-CD73 antibody, and the antibody further comprises sequences set forth in SEQ ID NO: 18, SEQ ID NO: 19 and SEQ ID NO: 20;
   (2) an isolated polypeptide, comprising sequences set forth in SEQ ID NO: 18, SEQ ID NO: 19 and SEQ ID NO: 20, wherein the polypeptide specifically binds to CD73 as a part of an anti-CD73 antibody, and the antibody further comprises sequences set forth in SEQ ID NO: 15, SEQ ID NO: 16 and SEQ ID NO: 17;
   (3) an isolated polypeptide, comprising a sequence set forth in SEQ ID NO: 2, SEQ ID NO: 6 or SEQ ID NO: 10, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence, wherein the polypeptide specifically binds to CD73 as a part of an anti-CD73 antibody, and the antibody further correspondingly comprises a sequence set forth in SEQ ID NO: 4, SEQ ID NO: 8 or SEQ ID NO: 12, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence;
   (4) an isolated polypeptide, comprising a sequence set forth in SEQ ID NO: 4, SEQ ID NO: 8 or SEQ ID NO: 12, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence, wherein the polypeptide specifically binds to CD73 as a part of an anti-CD73 antibody, and the antibody further correspondingly comprises a sequence set forth in SEQ ID NO: 2, SEQ ID NO: 6 or SEQ ID NO: 10, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence;
   (5) an isolated polypeptide, comprising a sequence set forth in SEQ ID NO: 10, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence, wherein the polypeptide specifically binds to CD73 as a part of an anti-CD73 antibody, and the antibody further correspondingly comprises a sequence set forth in SEQ ID NO: 14, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence; and
   (6) an isolated polypeptide, comprising the a sequence set forth in SEQ ID NO: 14, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence, wherein the polypeptide specifically binds to CD73 as a part of an anti-CD73 antibody, and the antibody further correspondingly comprises a sequence set forth in SEQ ID NO: 10, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence.
8. A nucleic acid molecule, encoding the antibody or the antigen-binding fragment thereof according to any one of paragraphs 1-6 or the isolated polypeptide according to paragraph 7.
9. A vector, comprising the nucleic acid molecule according to paragraph 8.
10. A host cell, comprising the nucleic acid molecule according to paragraph 8 or the vector according to paragraph 9.
11. A conjugate, comprising the antibody or the antigen-binding fragment thereof according to any one of paragraphs 1-6 and a conjugated moiety, wherein the conjugated moiety is a purification tag (for example, a His tag), a detectable label; preferably, the conjugated moiety is a radioisotope, a fluorescent substance, a chemiluminescent substance, a colored substance, polyethylene glycol or an enzyme.
12. A fusion protein or a multispecific antibody (preferably a bispecific antibody), comprising the antibody or the antigen-binding fragment thereof according to any one of paragraphs 1-6.
13. A kit, comprising the antibody or the antigen-binding fragment thereof according to any one of paragraphs 1-6, the conjugate according to paragraph 11, or the fusion protein or the multispecific antibody according to paragraph 12, wherein, preferably, the kit further comprises a secondary antibody specifically recognizing the antibody, optionally, the secondary antibody further comprises a detectable label, such as a radioisotope, a fluorescent substance, a chemiluminescent substance, a colored substance or an enzyme; and preferably, the kit is used for detecting the presence or level of CD73 in a sample.
14. A pharmaceutical composition, comprising the antibody or the antigen-binding fragment thereof according to any one of paragraphs 1-6, the conjugate according to paragraph 11, or the fusion protein or multispecific antibody according to paragraph 12, wherein, optionally, the pharmaceutical composition further comprises a pharmaceutically acceptable carrier and/or excipient, preferably, the pharmaceutical composition is in a form suitable for administration by subcutaneous injection, intradermal injection, intravenous injection, intramuscular injection or intralesional injection.
15. Use of the antibody or the antigen-binding fragment thereof of any one of paragraphs 1-6, the conjugate according to paragraph 11, or the fusion protein or multispecific antibody according to paragraph 12 in preparing drugs for treating and/or preventing tumors (such as solid tumors, preferably non-small cell lung cancer, prostate cancer (including metastatic castration-resistant prostate cancer (mCRPC)), triple-negative breast cancer, ovarian cancer, colorectal cancer (including colorectal cancer microsatellite stability (MSS)), gastric cancer, melanoma, head and neck cancer, renal cell carcinoma or pancreatic ductal adenocarcinoma), or in preparing a drug for diagnosing a tumor.
16. A hybridoma cell line, selected from:
   LT014, deposited at China Center for Type Culture Collection (CCTCC) with the accession number of CCTCC NO: C2018137.

## Claims

1. An anti-CD73 (for example, human CD73) antibody or an antigen-binding fragment thereof, wherein the anti-CD73 antibody comprises:
(1) a heavy chain variable region set forth in SEQ ID NO:2; and a light chain variable region set forth in SEQ ID NO:4,
(2) a heavy chain variable region set forth in SEQ ID NO:6; and a light chain variable region set forth in SEQ ID NO:8,
(3) a heavy chain variable region set forth in SEQ ID NO: 10; and a light chain variable region set forth in SEQ ID NO: 12,
wherein the antigen-binding fragment is selected from Fab, Fab', F(ab')₂, Fv, or Fab/c.

2. The anti-CD73 antibody or the antigen-binding fragment thereof according to claim 1, wherein the anti-CD73 antibody is IgG isotype and the antibody comprises a heavy chain constant region having a sequence set forth in SEQ ID NO: 21, and a light chain constant region having a sequence set forth in SEQ ID NO: 22; or the heavy chain constant region is Ig gamma-1 chain C region, ACCESSION: P01857, and the light chain constant region is Ig kappa chain C region, ACCESSION: P01834.

3. The anti-CD73 antibody or the antigen-binding fragment thereof according to any one of claims 1-2, wherein the antibody is a monoclonal antibody, a humanized antibody, a chimeric antibody or a multispecific antibody.

4. The anti-CD73 antibody or the antigen-binding fragment thereof according to any one of claims 1-3, wherein the antibody is a bispecific antibody.

5. The anti-CD73 antibody or the antigen-binding fragment thereof according to claim 2, wherein the antibody comprises (1) a heavy chain variable region having a sequence set forth in SEQ ID NO:6, a light chain variable region having a sequence set forth in SEQ ID NO:8, a heavy chain constant region having a sequence set forth in SEQ ID NO: 21, and a light chain constant region having a sequence set forth in SEQ ID NO: 22; or
(2) a heavy chain variable region having a sequence set forth in SEQ ID NO:10, a light chain variable region having a sequence set forth in SEQ ID NO:12, a heavy chain constant region having a sequence set forth in SEQ ID NO: 21, and a light chain constant region having a sequence set forth in SEQ ID NO: 22.

6. A nucleic acid molecule, encoding the antibody or the antigen-binding fragment thereof according to any one of claims 1-5.

7. A vector or a host cell, comprising the nucleic acid molecule according to claim 6.

8. A conjugate, comprising the antibody or the antigen-binding fragment thereof according to any one of claims 1-5 and a conjugated moiety, wherein the conjugated moiety is a purification tag (for example, a His tag) or a detectable label, preferably wherein the conjugated moiety is a radioisotope, a fluorescent substance, a chemiluminescent substance, a colored substance, polyethylene glycol or an enzyme.

9. A fusion protein or a multispecific antibody, preferably a bispecific antibody, comprising the antibody or the antigen-binding fragment thereof according to any one of claims 1-5.

10. A kit, comprising the antibody or the antigen-binding fragment thereof according to any one of claims 1-5, the conjugate according to claim 8, or the fusion protein or the multispecific antibody according to claim 9, wherein, preferably, the kit further comprises a secondary antibody specifically recognizing the antibody, optionally, the secondary antibody further comprises a detectable label, such as a radioisotope, a fluorescent substance, a chemiluminescent substance, a colored substance or an enzyme; and preferably, the kit is for detecting the presence or level of CD73 in a sample.

11. A pharmaceutical composition, comprising the antibody or the antigen-binding fragment thereof according to any one of claims 1-5, the conjugate according to claim 8, or the fusion protein or multispecific antibody according to claim 9, wherein, optionally, the pharmaceutical composition further comprises a pharmaceutically acceptable carrier and/or excipient, preferably, the pharmaceutical composition is in a form suitable for administration by subcutaneous injection, intradermal injection, intravenous injection, intramuscular injection or intralesional injection.

12. The antibody or the antigen-binding fragment thereof of any one of claims 1-5, the conjugate according to claim 8, the fusion protein or multispecific antibody according to claim 9, or the pharmaceutical composition according to claim 11, for use in treating and/or preventing tumors, preferably solid tumors.

13. The antibody or the antigen-binding fragment thereof of any one of claims 1-5, the conjugate according to claim 8, the fusion protein or multispecific antibody according to claim 9, or the pharmaceutical composition according to claim 11, for use in treating and/or preventing non-small cell lung cancer, prostate cancer, triple-negative breast cancer, ovarian cancer, colorectal cancer, gastric cancer, melanoma, head and neck cancer, renal cell carcinoma or pancreatic ductal adenocarcinoma.

14. The antibody, antigen-binding fragment, conjugate, fusion protein, multispecific antibody or pharmaceutical composition for use according to claim 13, wherein the prostate cancer is metastatic castration-resistant prostate cancer (mCRPC) or the colorectal cancer is microsatellite stable (MSS) colorectal cancer.

15. Use of the antibody or the antigen-binding fragment thereof of any one of claims 1-5, the conjugate according to claim 8, or the fusion protein or multispecific antibody according to claim 9, or the pharmaceutical composition according to claim 11, for diagnosing a tumor.
